Europäisches Patentamt

European Patent Office

Office européen des brevets

(11) Publication number: **0 313 537**
**A1**

## EUROPEAN PATENT APPLICATION

(21) Application number: **88850353.9**

(22) Date of filing: **20.10.88**

(51) Int. Cl.⁴: **A 61 K 31/135**
**C 07 C 93/06**

(30) Priority: **21.10.87 SE 8704081**

(43) Date of publication of application:
**26.04.89 Bulletin 89/17**

(84) Designated Contracting States:
**AT BE CH DE ES FR GB GR IT LI LU NL SE**

(71) Applicant: **Aktiebolaget Hässle**
**Kärragatan 5**
**S-431 83 Mölndal (SE)**

(72) Inventor: **Wikstrand, John Carl Magnus**
**Bangejordsgatan 13**
**S-412 73 Göteborg (SE)**

(74) Representative: **Näsman, Rune B. G. et al**
**AB Astra Patent and Trade Mark Department**
**S-151 85 Södertälje (SE)**

(54) **A new prophylactic method.**

(57) A method of improved prevention of death in a human patient having mild to moderate hypertension and no known previous history of myocardial infarction is described, said method comprising long-term daily administration of a prophylactically effective dosage of a pharmaceutically acceptable salt of metoprolol to said patient. Further described is use of metoprolol for manufacture of a pharmaceutical preparation for such improved prevention.

FIG. 3 TOTAL CUMULATIVE MORTALITY IN THE TWO RANDOMISATION GROUPS

EP 0 313 537 A1

**Description**

## A NEW PROPHYLACTIC METHOD

Technical field

The present invention is related to a new method of therapy in the cardiovascular field and to use of a known pharmaceutical in manufacture of a preparation for a new therapeutic application.

Background art

Mortality is increased in patients with high blood pressure. Although a number of drugs reduce blood pressure, so far only thiazide diuretics have been demonstrated to reduce mortality in large-scale, well-controlled scientific studies.

Current evidence suggests that antihypertensive treatment is beneficial for patients with diastolic blood pressure (DBP) persistently above 100 mm Hg as well as for those with lesser elevation but who are also at risk, such as those with end-organ damage, or other major risk factors for coronary heart disease. The current debate is about what antihypertensive agent is most suitable as initial treatment for mild to moderate hypertension.[1,2]

Guidelines published by the Joint National Committee on High Blood Pressure in the USA, and by other similar bodies in most countries throughout the world, recommend starting treatment of mild to moderate hypertension with either a thiazide diuretic or a β-blocker.[2,3] Several well-controlled studies have shown that these drugs have an equal blood pressure-lowering effect and are well tolerated.[2] Although subjective symptoms may differ somewhat with the two types of drug,[4] side effects are usually mild and few. Studies have also shown the efficacy and safety of both β-blockers and diuretics in elderly patients.[5]

The choice between these two first-line drugs must take into consideration not only their anti-hypertensive efficacy and tolerability but also, and most importantly, their long-term effects on the cardiovascular complications of high blood pressure. One early non-randomised trial, using β-blockade as initial treatment, indicated a beneficial effect on coronary heart disease (CHD, non-fatal and fatal myocardial infarction and sudden cardiac death).[6,7] The results of this trial together with the results from an early post-infarction study with a β-blocker,[8] formed the basis for planning and conducting the study,[9] which resulted in the present invention.

Said primary preventive study aimed at investigating whether metoprolol, a relatively β₁-selective β-blocker, given as initial antihypertensive treatment, would lower cardiovascular complications of high blood pressure to a greater extent than do thiazide diuretics as initial treatment.

Description of the invention

Metoprolol, a relatively β₁-selective β-blocker, has been compared with thiazide diuretics as initial antihypertensive treatment in a large scale well controlled international study. Metoprolol reduced total mortality better than thiazide diuretics in spite of very similar blood pressure control. This unexpected finding on total mortality was explained by a reduction in cardiovascular deaths, deaths due to heart attacks and stroke with no adverse effects on non-cardiovascular mortality. By total mortality is meant the total number of deaths, without restriction to cause of death, in a study population of patients. By cardiovascular mortality is meant the number of deaths in cardiovascular disease in a study population of patients, as further described below under the heading "Classification of cause-specific mortality".

Thus, the present invention defines a method of improved primary prevention of death of a human patient having mild to moderate hypertension and no known previous history of myocardial infarction, brain infarction or cerebral hemorrhage said method comprising long-term daily administration of a prophylactically effective dosage of a metoprolol or a pharmaceutically acceptable salt thereof to said patient. The word prevention in this context must of course, be understood in a relative rather than absolute sense in that the prevention of death in cardiovascular disease in individual patients may be more or less successful and in that a preventing effect on death by other causes is not seen.

While it is an unexpected finding of the invention that mortality is reduced in patients having no known history of myocardial infarction, brain infarction or cerebral hemorrhage, it is an even more unexpected finding that mortality is reduced in patients having no known history of severe cardiovascular disease, such as angina pectoris, and in particular in patients merely having uncomplicated mild to moderate hypertension.

In a further aspect the present invention is related to use of metoprolol as an active ingredient in manufacture of a pharmaceutical preparation for improved prevention of death in a human patient having mild to moderate hypertension and no known previous history of myocardial infarction, brain infarction or cerebral hemorrhage by long-term daily administration of a prophylactically effective dosage of such preparation. Such use is in particular for manufacture of a drug for treatment of a patient having a history as just described. Manufacture as such is carried out by methods known in the art.

A prophyllactically effective dosage of racemic metoprolol tartrate in an average weighted adult is 100 to 200 mg per day. However, dosages outside said range, and in particular dosages in the range of 50 to 400 mg per day, are within the scope of the invention. Pharmaceutically acceptable salts of metoprolol include for example the tartrate, the fumarate and the succinate.

Below is a description of the study behind the present invention.

PATIENTS AND METHODS

Study population

The study included 3234 patients, 1609 randomised to a metoprolol group and 1625 to a diuretic group. Patients were recruited from 66 clinical centres in 11 countries.

Both previously treated patients and those with newly detected and untreated hypertension were eligible for inclusion in the study. All were men out-patients aged 40 to 64 years. The patients' diastolic blood pressure in the sitting position was $\geq$100 mm Hg and <130 mm Hg at randomisation, calculated as the mean of two readings taken 2 weeks apart and including two readings on each occasion. Since this was a study of primary prevention previous myocardial infarction, angina pectoris and stroke were exclusion criteria. Further exclusion criteria were secondary hypertension, second- and third-degree atrio-ventricular block, cardiac failure, obstructive lung disease not well controlled by $\beta_2$-stimulants, diabetes mellitus (defined as two separate positive dip tests for glucosuria and a fasting blood glucose level above 6.8 mmol/1), gout, and serious diseases such as malignant disease or severe alcoholism.

Study design

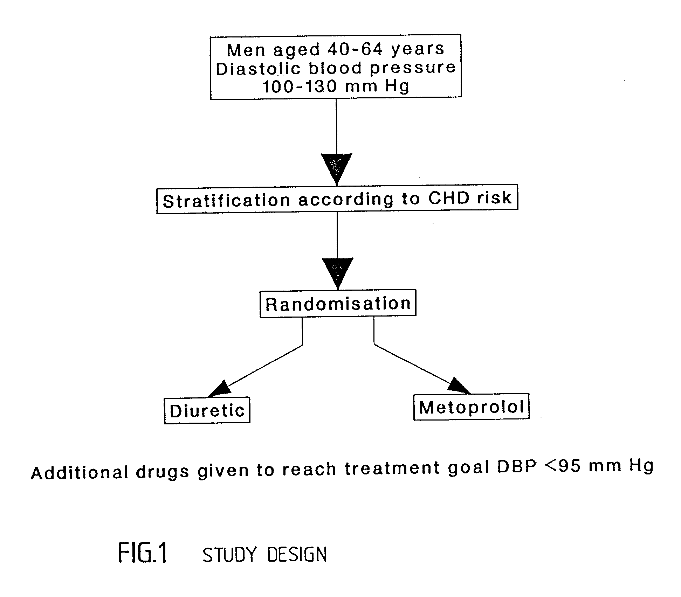

This was an international, multicentre, randomised, stratified, open, controlled study of primary prevention in parallel groups preceded, for previously treated patients, by a four to eight week run-in period without treatment (Fig 1). The majority of patients included were previously untreated and found by screening procedures.

Before randomisation, patients were stratified according to risk of CHD into nine risk groups depending on age (40-49; 50-59; $\geq$60, years), first systolic BP (SBP) recorded (<190; $\geq$190 mm Hg), cholesterol level (<7.0; 7.0-8.6; $\geq$8.7 mmol/l), and smoking habits (never smoked; ex-smoker; 1-14; $\geq$15, gram/day where 1 cigarette = 1 gram). An ex-smoker was defined as having stopped smoking at least one month previously.

After stratification, patients were randomised to treatment with either metoprolol 200 mg daily or a thiazide diuretic (hydrochlorothiazide 50 mg, or bendroflumethiazide 5 mg daily).

The dose was doubled or additional drugs (hydralazine, spironolactone or others, but not $\beta$-blockers or thiazide diuretics) were given, if necessary, to reach the treatment goal of DBP <95 mm Hg (9). In a later phase of the study, the maximum dosages of the baseline drugs were reduced to 200 mg of metoprolol or 50 mg of hydrochlorothiazide (5 mg bendroflumethiazide).

According to protocol criteria stated in an addendum to the protocol, a patient in the diuretic group could receive $\beta$-blockade if the physician considered this to be indicated for clinical reasons (ie in some patients suffering a non-fatal myocardial infarction during the study).

Methods

Blood pressure recordings were taken after 5 minutes' rest twice at an interval of 15 minutes. Blood pressure was measured blindly (Hawksley random-zero sphygmomanometer or London School of Hygiene blind manometer) in the right arm with the patient in the sitting position.

The following laboratory investigations were performed: measurement of serum cholesterol, serum potassium, serum uric acid and serum creatinine; dip tests for glucosuria and albuminuria; and a twelve lead resting ECG. Body weight and height were also measured.

Compliance with the treatment to which the patient had been randomised was judged by asking all investigators to specify at the last follow-up visit all drugs and dosages used. Data on drug compliance was given for those patients followed for the whole study period, separately for the group of patients followed up to the median (4.16 years) and for the group with a follow-up time >6.15 years (=upper quartile).

Patients were included only on giving consent after full explanation of the nature and purpose of the investigation.

Methodological considerations

Initially most centres randomised patients either to metoprolol or to a thiazide diuretic, although the original protocol also permitted the $\beta$-blockers propranolol and alprenolol. Later the protocol was changed so that centres wishing also to randomise patients to atenolol or to a thiazide diuretic could take part in the study.

It became obvious that the trial would reach more than 20 000 patient years it was formally decided to close the trial at the time that such number would be reached.

However, 66 of the 70 investigators at centres using metoprolol decided to continue the follow-up of all patients after close of the original trial. The aim with this follow-up was to obtain further data regarding the possible cardiovascular protective benefits of metoprolol. No information whatsoever was available about end-points at the time the decision was taken to extend the study. However, results from four double-blind studies of intervention with metoprolol in patients with myocardial infarction were available: the Gothenburg Metoprolol trial, the Amsterdam Metoprolol trial, the Belfast Metoprolol trial, and the Stockholm Metoprolol trial.[10,11,12,13] In the pooled results from these double-blind placebo controlled trials it was evident that metoprolol, in comparison with placebo, reduced total mortality by approximately 25% (123,1558 vs 161/1521, p<0.008) and sudden death by 50% (35/1558 vs 69/1521, p<0.001) subsequent to myocardial infarction.

3

Furthermore, in the Stockholm Metoprolol trial other kinds of non-fatal atherosclerotic events were also reduced in the metoprolol group: cerebrovascular events, need for coronary artery by-pass surgery because of disabling angina pectoris, and lower limb amputation because of peripheral arterial insufficiency.[13] The effect on non-fatal atherosclerotic complications was especially obvious in patients with a history of hypertension.[14] Taken together these data formed the basis for decision to perform the follow-up in centres.

Towards the end of the metoprolol follow-up study, data on compliance were presented. Of special interest was, of course, the use of β-blockade in patients randomised to diuretics, for if a cardioprotective effect of the β-blocker exists, this type of cross-over will diminish the possibility of proving such an effect. The data showed an increasing use of β-blockade in the diuretic group as the follow-up period lengthened (Table 1). It was therefore decided to close the study.

Follow-up

Every patient entered on the randomisation list was included in the follow-up, regardless of treatment status. The start of treatment was defined as the date of randomisation. For living patients, the end of the study was defined as the day of the last follow-up interview.

Administration of case record forms and follow-up

All information on whether patients were alive, dead or lost to follow-up was gathered by using a number of specially designed questionnaires. A specially designed questionnaire was also sent to all investigators who reported that patients had died during the follow-up asking for detailed information on death certificates, hospital files, and necropsies.

A major operation was started, to try to trace the 152 patients reported at that time as lost to follow-up. At the time of last information only one patient was still lost to follow-up - a patient randomised in the UK who had moved to Uganda.

At the closure of the study, 3085 patients were reported alive, 148 dead and one lost to follow-up (randomised to diuretic). Of the 3085 alive, 3039 were seen by the investigators, 27 contacted personally by telephone, 3 reported alive by relatives and 16 by up-to date population register.

When an Independent End-point Committee classified the 148 deaths during the second half of July 1987, death certificates were available for 115 patients, hospital based files but no death certificate for 17, signed letters from investigators defining identity and circumstances of death for 9, and other information, such as police reports and non-hospital based medical files, for 7 patients.

Classification of cause-specific mortality

The cause of death was defined by using all available information such as hospital records, physicians' reports, police reports, death certificates and necropsies. If the death occurred within 28 days of the onset of an event, that event was classified as the fatal event - otherwise the event was classified as non-fatal, and a subsequent event, if fatal, was coded as the cause of death. A decision regarding cause of death was made by the Independent End-point Committee on each of the 148 deaths. All cases were judged without any knowledge of actual treatment or of which treatment the patient had originally been randomised to. These data were also subsequently scrutinised in each single case, and approved by an Independent Data Audit Committee also without any information as to actual treatment or randomisation group.

STATISTICAL METHODS

All data were analysed by computer techniques, utilising the SAS® program.[15] With the Gehan-Wilcoxon non-parametric test for survival analysis the null hypothesis was tested, ie that there was no difference between the two treatments (intention to treat) in death rates.[15,16]

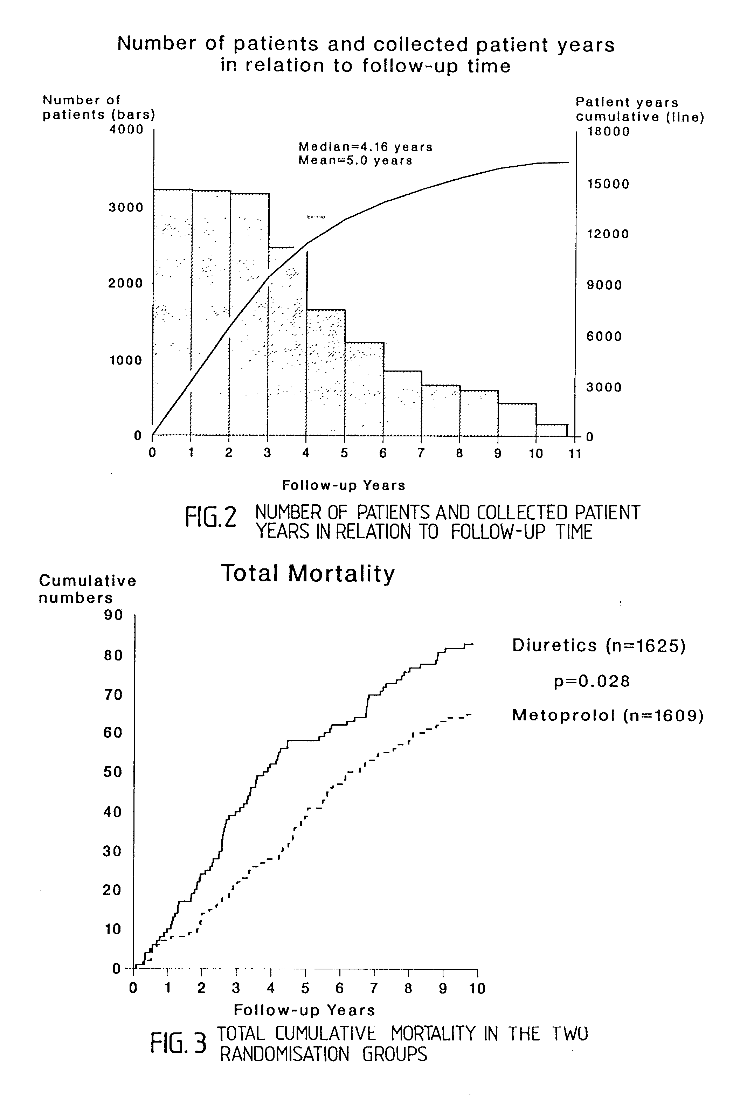

Survival analysis methods are necessary in trials where subjects are entered over a period of time and have variable lengths of follow-up. These methods permit comparison of the entire survival results during the follow-up.[16] In an attempt to illustrate also the extent of the possible difference in effect between the two treatment regimens, confidence limits were calculated at the time of median follow-up, 4.16 years. This point in time was chosen since results from a large number of patients were gathered then (see Figure 2).

For quantitative variables regarded as normally distributed, ie, blood pressure and heart rate, parametric tests were used (Student's t-test). Differences in proportions were tested by Fisher's exact test. p values below 0.05 (two-sided tests) were considered significant for all variables.

RESULTS

Length of follow-up

All patients were followed for at least 842 days or until they died. The median follow-up time was 4.16 years, and the mean follow-up time 5.00 years (Fig 2). The longest follow-up in any patient was 10.8 years. In total, 16 180 patient years were collected, 8 109 in patients randomised to metoprolol, 8 071 years in patients randomised to diuretics.

Compliance

Table 1 shows antihypertensive treatment at the last follow-up visit, at the median follow-up time (4.16 years), and in those followed for more than 6.15 years (upper quartile). The mean dose of β-blockade in patients randomised to metoprolol was 174 mg daily and the mean dose of hydrochlorothiazide and bendroflumethiazide in patients randomised to diuretics was 46 mg and 4.4 mg daily, respectively. At the last follow-up visit, 11.6 % of patients randomised to diuretics were reported to be receiving β-blockade, the corresponding figure for use of diuretics in the β-blocker group was 6.3 %. Of patients with a follow-up time equal to or shorter than the median (4.16 years), 6.6 % of the diuretic group were receiving β-blockade, compared with 20.4 % of diuretic treated patients followed for more than 6.15 years (upper quartile). The corresponding figures for use of diuretics in the β-blocker group were 4.6 % and 6.9 % (Table 1).

Clinical characteristics at randomisation and during follow-up

The two randomisation groups were well matched in major clinical characteristics at entry as well as at the last follow-up visit (Table 2). As expected, heart rate was lower with β-blockade, and serum potassium lower, and serum uric acid concentration higher in patients randomised to diuretics at follow-up. There was an increase in body weight in patients randomised to β-blockade (1.5 kg/5.1 years). Both groups showed a slight decrease in percentage of patients with albuminuria (in absolute figures -1.7 % for patients randomised to β-blockade, -1.2 % for patients randomised to diuretics) and a slight increase in glucosuria (+0.8 % and +1.2 %, respectively). There was a slight statistically significant decrease in serum cholesterol in the metoprolol group. Approximately 10 % of the patients in each group stopped smoking. In subgroups of non-smokers and smokers clinical characteristics at entry as well as at last follow-up visit were similar to those in the whole study population. Blood pressure at randomisation in non-smokers randomised to metoprolol was 166.2/107.4 mm Hg, in smokers 168.2/108.1 mm Hg, at last follow-up 142.3/88.6 mm Hg and 142.7/89.0 mm Hg, respectively. The corresponding values for patients randomised to diuretics were 165.7/107.2 and 169.0/108.0 mm Hg at entry; 142.3/89.5 and 143.6/89.7 mm Hg at last follow-up visit, respectively.

Total mortality

Total mortality was significantly lower in patients randomised to metoprolol than in patients randomised to diuretics (p=0.028, Fig 3, Table 3). Table 3 gives figures for total, cardiovascular and non-cardiovascular mortality at the shortest follow-up time for all patients (842 days), at the median follow-up time (4.16 years) and at the end of the study. The 95 % confidence limit for the difference in total mortality in patients randomised to metoprolol compared with those randomised to diuretics at median follow-up time (4.16 years) was -68 % to -17 %.

Although the mortality rates (deaths/1000 patient years) differed substantially between different populations, the percentage difference in mortality between patients randomised to metoprolol and to diuretics seemed to be approximately the same in different geographical regions. The relative benefit was similar in centres comparing metoprolol with hydrochlorothizide and in centres comparing metoprolol with bendroflumethiazide.

Cause-specific mortality

Significantly lower cardiovascular mortality (p=0.012), coronary heart disease mortality (p=0.048) and stroke mortality (p=0.043) were observed in patients randomised to metoprolol (Table 4). Three patients randomised to β-blockade died of severe heart failure, one with a severe aortic stenosis and the other two after large non-fatal myocardial infarctions.

Total mortality in non-smokers and smokers

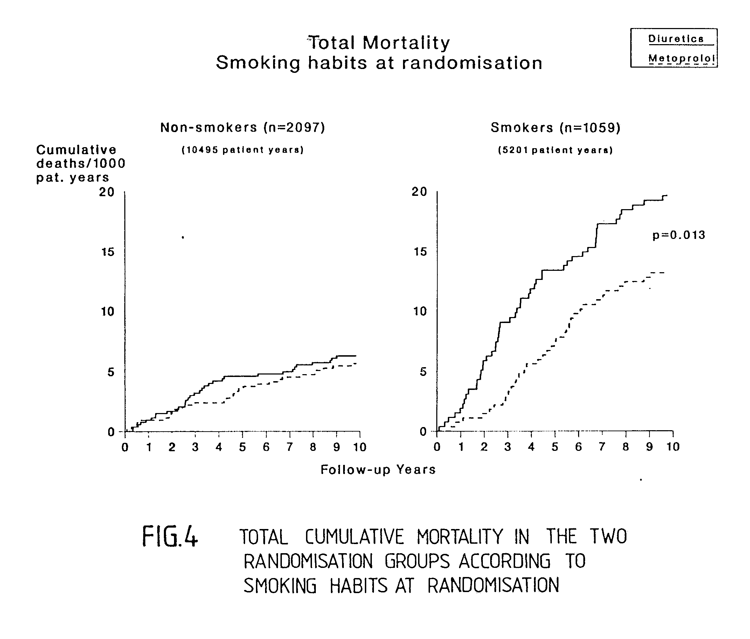

Total mortality was significantly lower in smokers randomised to metoprolol than in smokers randomised to diuretics (p=0.013, Fig 4, Table 5). There was also a tendency towards somewhat fewer deaths in non-smokers randomised to metoprolol. Table 5 gives figures separately for non-smokers and smokers for total mortality at the shortest follow-up time for all patients (842 days), at the median follow-up time (4.16 years) and at the end of the study.

COMMENTS

The study showed that total and cardiovascular mortality was lower in patients randomised to the metoprolol regimen than in those randomised to the diuretic regimen. At the time of median follow-up the difference in total mortality was 48 % in favour of patients randomised to metoprolol, with a 95 % confidence limit ranging between -68 % and -17 %. The decreasing difference in mortality between treatment groups with increasing follow-up might be explained partly by the increase in treatment cross-overs, especially the increasing use of β-blockade in the diuretic group; partly by an increasing number of drop-outs from randomised treatment; and also by an increase in the number of non-cardiovascular deaths in both groups. Furthermore, β-blockade probably delays an end-point in many patients, rather than preventing it completely (see below).

Baseline characteristics were similar in the two treatment regimens. There was a highly significant reduction in systolic and diastolic blood pressure in both groups, to a similar level. Thus, the difference in mortality was due to factors other than differences in achieved blood pressure control. Analysis of cause-specific mortality showed that the reduction in mortality was mainly due to a reduction in fatal coronary heart disease and fatal stroke.

The pathophysiological mechanisms behind the beneficial action of metoprolol on cardiovascular complications shown in this primary preventive study, and in earlier secondary preventive studies (see Methodological considerations) are not known.

Effects on flow disturbances as well as effects on the cellular level have been suggested.[17-23] One could speculate on an effect which changes the triggering level for the occurrence of an end-point, on a deceleration of the progression rate of the underlying disease, or both.

The open study design can be discussed. However, when planning the study a double-blind design was not considered feasible in an international long-term study of this size and nature. The advantage of the present design is that it mirrors everyday clinical practice. The stratification and randomisation procedures, together with the results of baseline characteristics, the blind evaluation of hard end-points, and the completeness of follow-up make it unlikely that there is any significant bias stemming from the study design.

In two earlier primary preventive studies published in 1985, the MRC and IPPPSH studies, no differences in mortality were detected between a β-blocker based (propranolol, non-selective in the MRC and oxprenolol, non-selective with ISA, in the IPPPSH study) and a non-β-blocker, mainly diuretic-based, regimen.[24,25] Also the results of the original phase of the present study, including other β-blockers were non-conclusive in this respect.[26]

There are no obvious explanations for the differences in results between these major well-controlled, randomised studies. One could speculate that technical problems such as a higher drop-out rate, or lower compliance, or both could at least partly play a role in the other studies. The completeness of follow-up in the metoprolol study is also a factor that could be of importance. A major effort was made to define the vital status of all randomised patients reported to be lost to follow-up. When mortality was analysed subsequent to the study only one patient was still not traced. Whatever the status of that patient the results and conclusions of this study remain unaffected since this patient was randomised to diuretics.

One further factor that could be of importance is sex. The metoprolol study included only men, while the MRC and IPPPSH studies recruited both men and women. In women, low rates of events, particularly for CHD and total mortality, substantially reduce the likelihood of detecting any reduction in event rate attributable to the treatment under study.[27]

A striking effect was shown in smokers in the present study, with a significantly lower total mortality in patients randomised to metoprolol. Different results were found in the MRC and IPPPSH studies in which subgroup analysis showed a lower mortality in non-smoking men randomised to β-blockade, but not in smokers. Caution is of course necessary when interpreting sub-group analysis, and it cannot be ruled out that these differences in event rates occurred by chance. It is important to note in this respect that no stratification was done for smoking in the MRC and IPPPSH studies, in contrast to the metoprolol study. The likelihood that results in non-smokers are chance findings in the IPPPSH study is supported by the unexpected finding that very little difference in mortality was seen between non-smoking and smoking men randomised to diuretics, because of an unexpectedly high mortality in non-smokers. It is clear from baseline and follow-up characteristics in the present study that the stratification was successful. This conclusion is further strengthened by the expected three-fold increase in mortality in smokers as compared with non-smokers in the group randomised to diuretic treatment.

Another factor of importance when interpreting the results in smokers is the type of β-blocker used. Metoprolol is a relatively β₁-selective β-blocker in contrast to the non-selective β-blockers, propranolol and oxprenolol. Smoking will lead to an increase in adrenaline,[28] which in smokers on non-selective β-blockade might lead to an unopposed α-constriction in peripheral resistance vessels interfering with BP control.[29] In the MRC study blood pressure was higher in β-blocker treated smokers than in those treated with diuretics while in the present study blood pressure control was equally effective regardless of smoking habits at randomisation.

This study did not aim at detecting differences in unwanted subjective symptoms between the two regimens. Other studies specifically designed to tackle this question showed that there were no important differences between a metoprolol and a thiazide diuretic regimen.[5] The metoprolol regimen seems to be safe judging by the similar rate of non-cardiovascular deaths in the two randomisation groups.

In conclusion, starting antihypertensive therapy in men with mild to moderate hypertension with the β₁-selective β-blocker metoprolol instead of a thiazide diuretic, leads to a lower total and cardiovascular mortality, mainly by reducing fatal CHD and fatal stroke. The demonstrated benefit on total mortality, ranging between -68 % to -17 % compared with diuretics (95 % confidence limits) at median follow-up time after 4.2 years, seems to have important implications for clinical practice and public health policy because of the high prevalence of hypertension and the increased risk in those with hypertension.

REFERENCES

1. Chobanian AV: Treatment of mild hypertension - the debate intensifies. J Cardiovasc Med 1983;8:883-888.

2. Wikstrand J: Initial therapy for mild hypertension. Pharmacotherapy 1986;6(2):64-72.

3. Joint National Committee on Detection, Evaluation and Treatment of High Blood Pressure. 1984 report. Arch Intern Med 1984;144:1045-1057.

4. Medical Research Council Working Party on Mild to Moderate Hypertension: Adverse reactions to bendrofluazide and propranolol for the treatment of mild hypertension. Lancet 1981;ii:539-543.

5. Wikstrand J, Westergren G, Berglund G, et al: Antihypertensive treatment with metoprolol or hydrochlorothiazide in patients aged 60 to 75 years: Report from a double-blind international multicenter study. JAMA 1986;255:1304-1310.

6. Berglund G, Wilhelmsen L, Sannerstedt R, et al: Coronary heart disease after treatment of hypertension. Lancet 1978;1:1-5.

7. Andersson O, Berglund G, Hansson L, et al: Organization and efficacy of an out-patient hypertension clinic. Acta Med Scand 1978;203:391-398.

8. Wilhelmsson C, Wilhelmsen L, Vedin JA, G Tibblin, Werkö L: Reduction of sudden deaths after myocardial infarction by treatment with alprenolol. Lancet 1974;ii:1157-1160.

9. Wilhelmsen L, Berglund G, Elmfeldt D, et al: β-Blockers versus saluretics in hypertension. Comparison of total mortality, myocardial infarction and sudden death: Study design and early results on blood pressure reduction. Prev Med 1981; 10:38-49.

10. Hjalmarson Å, Herlitz J, Holmberg S, et al: The Göteborg metoprolol trial: Effects on mortality and morbidity in acute myocardial infarction. Circulation 1983;67:126-132.

11. Manger Cats V, van Capelle FJL, Lie KI, Durrer D: The Amsterdam metoprolol trial effect of treatment with metoprolol on first-year mortality in a single-centre study with low placebo mortality rate after myocardial infarction. Drugs 1985;29(suppl 1):8.

12. Salathia KS, Barber JM, McIlmoyle EL, Nicholas J, Evans AE, Elwood JH, Cran G, Shanks RG, Boyle D McC: Very early intervention with metoprolol in suspected acute myocardial infarction. Eur Heart J 1985;6:190-198.

13. Olsson G, Rehnqvist N, Sjögren A, Erhardt L, Lundman T: Long-term treatment with metoprolol after myocardial infarction: Report on three year mortality and morbidity. J Am Coll Cardiol 1985;5:1428-1437.

14. Olsson G, Rehnqvist N. Reduction of non-fatal reinfarctions in patients with a history of hypertension by chronic postinfarction treatment with metoprolol. Acta Med Scand 1986;220:33-38.

15. SAS® User's Guide. Cary, NC: SAS Institute Inc, 1983.

16. Friedman L, Furberg C, DeMets D: Fundamentals of clinical trials. John Wright. PSG Inc, Boston, Bristol, London, 1983, pp 174-194.

17. Åblad B, Björkman J-A, Edvardsson N, Hansson GA: Betaadrenergic mechanism in the arterial wall possibly involved in genesis of cardiovascular disease. Abstract. Fed Proc 1987;46:

18. Kaplan JR, Manuck SB, Clarkson TB, Adams MR, Weingand KW: Effects of propranolol HCl on behaviorally induced atherosclerosis in hyperlipoproteinemic monkeys. Abstract. Arteriosclerosis 1986;6:545a.

19. Chobanian AV: The influence of hypertension and other hemodynamic factors in atherogenesis. Prog in Cardiovasc Dis 1983;XXVI No 3:177-195.

20. Bell FP: Effects of antihypertensive agents propranolol, metoprolol, nadolol, prazosin and chlorthalidone on ACAT activity in rabbit and rat aortas and on LCAT activity in human plasma in vitro. J Cardiovasc Pharmacol 1985;7:437-442.

21. Spence JD: Hemodynamic effects of antihypertensive drugs. Possible implications for the prevention of atherosclerosis. Hypertension 1984;6:163-168.

22. Chobanian AV, Brecher P, Chan C: Effects of propranolol on atherogenesis in the cholesterol-fed rabbit. Circ Res 1985;56:755-762.

23. Spence JD: Antihypertensive drugs and prevention of atherosclerotic stroke. Stroke 1986;17:808-810.

24. Medical Research Council Working Party: MRC trial of treatment of mild hypertension: Principal results. Br Med J 1985;291:97-104.

25. The IPPPSH Collaborative Group: Cardiovascular risk and risk factors in a randomized trial of treatment based on the beta- blocker oxprenolol: The International Prospective Primary Prevention Study in Hypertension (IPPPSH). J Hypertension 1985;3:379-392.

26. Wilhelmsen L, Berglund G, Elmfeldt D et al: Beta-blockers versus diuretics in hypertensive men: Main results from the HAPPHY trial. J Hypertension (in press).

27. MacMahon S, Cutler JA, Furberg D, Payne G: The effects of drug treatment for hypertension on morbidity and mortality from cardiovascular disease: A review of randomized controlled trials. Prog Cardiovasc Dis 1986; XXIX No 3(suppl 1):99-118.

28. Cryer PE, Haymond MW, Santiago JV, Shah SD: Norepinephrine and epinephrine release and adrenergic mediation of smoking-associated haemodyamic and metabolic events. N Engl J Med 1976;295:573-577.

29. Trap-Jensen J, Carlsen JE, Svendsen TL, Christensen NJ: Cardiovascular and adrenergic effects of cigarette smoking during immediate non-selective and selective beta adrenoceptor blockade in humans. Eur J Clin Invest 1979;9:181-183.

LE GENDS TO FIGURES

Figure 1 Study design.

Figure 2 Number of patients and cumulative patient years in relation to follow-up time.

Figure 3 Total cumulative mortality in the two randomisation groups.

Figure 4 Total cumulative mortality in the two randomisation groups according to smoking habits at randomisation.

Table 1. Treatment status at last follow-up in relation to time of follow-up. Values within broken horizontal lines in the left-hand side of the table give the reported use of diuretics in patients randomised to metoprolol; in the right-hand side the reported use of ß-blockade in patients randomised to diuretics.

| | Randomised to | | | | | | | | | | | |
| | METOPROLOL | | | | | | DIURETIC | | | | | |
| | all | | follow-up time median <4.16 years | | 4th quartile >6.15 years | | all | | follow-up time median <4.16 years | | 4th quartile >6.15 years | |
| Antihypertensive treatment | % | cumu-lative % | % | cumu-lative % | % | cumu-lative % | % | cumu-lative % | % | cumu-lative % | % | cumu-lative % |
|---|---|---|---|---|---|---|---|---|---|---|---|---|
| Including ß-blockade | | | | | | | | | | | | |
| ß-blockade monotherapy | 51.9 | 51.9 | 50.3 | 50.3 | 54.1 | 54.1 | 4.7 | 4.7 | 2.8 | 2.8 | 8.0 | 8.0 |
| ß-blockade + other, no diuretic | 28.8 | 80.7 | 31.8 | 82.1 | 25.1 | 79.3 | 2.3 | 7.0 | 1.5 | 4.3 | 3.7 | 11.7 |
| ß-blockade + diuretic + no other | 2.0 | 82.7 | 1.1 | 83.2 | 2.7 | 82.0 | 3.1 | 10.1 | 1.8 | 6.1 | 5.2 | 17.0 |
| ß-blockade + diuretic + other | 1.1 | 83.8 | 0.6 | 83.9 | 1.7 | 83.7 | 1.5 | 11.6 | 0.5 | 6.6 | 3.5 | 20.4 |
| Not including ß-blockade | | | | | | | | | | | | |
| Diuretic monotherapy | 2.0 | 85.8 | 1.8 | 85.6 | 1.5 | 85.1 | 45.4 | 57.0 | 45.7 | 52.2 | 42.9 | 63.3 |
| Diuretic + other, no ß-blockade | 1.2 | 86.9 | 1.1 | 86.7 | 1.0 | 86.1 | 28.1 | 85.1 | 34.3 | 86.5 | 20.7 | 84.0 |
| Other, no ß-blockade, no diuretic | 2.9 | 89.9 | 3.6 | 90.4 | 2.2 | 88.3 | 3.7 | 88.8 | 3.5 | 90.0 | 3.7 | 87.8 |
| Off any treatment | 6.5 | 96.3 | 5.3 | 95.6 | 8.8 | 97.1 | 6.9 | 95.7 | 4.7 | 94.8 | 10.0 | 97.8 |
| No information available | 3.7 | 100 | 4.4 | 100 | 2.9 | 100 | 4.3 | 100 | 5.2 | 100 | 2.2 | 100 |

EP 0 313 537 A1

Table 2.

Clinical characteristics of patients at entry and at last follow-up visit (n = 3234).

| | At entry Randomised to | | At last follow-up Randomised to | |
| --- | --- | --- | --- | --- |
| | metoprolol (n = 1609) | diuretic (n = 1625) | metoprolol | diuretic |
| Means ± SD | | | | |
| SBP (mm Hg) | 166.8 ± 17 | 166.8 ± 17 | 142.7 ± 17 | 142.7 ± 16 |
| DBP (mm Hg) | 107.6 ± 6 | 107.5 ± 6 | 88.7 ± 8 | 89.5 ± 8* |
| HR (beats/min) | 78.2 ± 10 | 77.3 ± 10 | 64.1 ± 10 | 74.1 ± 11** |
| Age (years) | 52.6 ± 7 | 52.6 ± 7 | 57.7 ± 7 | 57.6 ± 7 |
| Body weight (kg) | 83.2 ± 12 | 82.7 ± 12 | 84.7 ± 12 | 82.6 ± 12** |
| BMI (kg/m²) | 27.3 ± 3.5 | 27.1 ± 3.5 | 27.6 ± 3.7 | 26.9 ± 3.5** |
| S-Cholesterol (mmol/l) | 6.32 ± 1.2 | 6.29 ± 1.2 | 6.15 ± 1.2 | 6.32 ± 1.2** |
| S-Creatine (umol/l) | 93.1 ± 17 | 93.1 ± 16 | 97.7 ± 19 | 96.8 ± 16 |
| S-Potassium (mmol/l) | 4.28 ± 0.4 | 4.27 ± 0.4 | 4.28 ± 0.4 | 3.97 ± 0.4** |
| S-Urate (umol/l) | 349 ± 76 | 347 ± 80 | 364 ± 75 | 384 ± 82** |
| Proportions % | | | | |
| S-Potassium < 3.6 mmol/l | 5.2 | 6.3 | 1.7 | 13.0** |
| S-Urate > 450 umol/l | 9.2 | 9.0 | 11.9 | 18.2** |
| Glucosuria | 1.5 | 0.4 | 2.3 | 1.6 |
| Albuminuria | 4.7 | 3.9 | 3.0 | 2.7 |
| Smokers | 34 | 33 | 23 | 25 |
| Major Q-wave[a] | 2.1 | 1.8 | - | - |

*p = 0.012;
**p 0.001 between group comparion at last follow-up visit.
[a]1:1 or 1:2 according to Minnesota coded ECG.

SBP = Systolic blood pressure. D = diastolic. HR = Heart rate.

BMI = Body mass index. S = serum.

Table 3. Total, cardiovascular and non-cardiovascular mortality in the whole study population at the shortest follow-up time for all patients (842 days), at the median follow-up time (4.16 years) and at end of follow-up.

| | Total mortality | | | | | Cardiovascular mortality | | | | | Non-cardiovascular mortality | | | | |
| | Randomised to | | | | | Randomised to | | | | | Randomised to | | | | |
| | metoprolol | | diuretic | | Differ-ence | metoprolol | | diuretic | | Differ-ence | metoprolol | | diuretic | | Differ-ence |
| Follow-up time | n | rate* | n | rate* | % | n | rate* | n | rate* | % | n | rate* | n | rate* | % |
| 842 days | 15 | 4.1 | 27 | 7.3 | −44 | 7 | 1.9 | 19 | 5.1 | −63 | 8 | 2.2 | 8 | 2.2 | 0 |
| Median (4.16 years) | 28 | 4.8 | 54 | 9.3 | −48 | 15 | 2.6 | 36 | 6.2 | −58 | 13 | 2.2 | 18 | 3.1 | −29 |
| End of study | 65 | 8.0 | 83 | 10.3 | −22 | 42 | 5.2 | 57 | 7.1 | −27 | 23 | 2.8 | 26 | 3.2 | −13 |

*rate = cases/1000 patient years

EP 0 313 537 A1

Table 4.

Cause-specific mortality.

| | Randomised to | | 2p |
|---|---|---|---|
| | metoprolol (n = 1609) | diuretic (n = 1625) | |
| Cardiovascular | 42 | 57 | 0.012 |
| Fatal CHD (MI or SD coronary) | 36 | 43 | 0.048 |
| SD, pulmonary embolism | 0 | 3 | |
| Fatal stroke | 2 | 9 | 0.043 |
| Aortic aneurysm | 1 | 2 | |
| Heart failure | 3 | 0 | |
| Non-cardiovascular | 23 | 26 | |
| Cancer | 16 | 18 | |
| Suicide | 3 | 3 | |
| Accident | 1 | 4 | |
| Infection | 2 | 0 | |
| Other | 1 | 1 | |
| Total mortality | 65 | 83 | 0.028 |

CHD = coronary heart disease.
MI = myocardial infarction. SD = sudden death

Table 5.

Total mortality in non-smokers and smokers at the shortest follow-up time for all patients (842 days), at the median follow-up time (4.16 years) and at the end of the study.

| Follow-up time | Non-smokers (n=2097) | | | | | Smokers (n=1059) | | | | |
| | Randomised to | | | | | Randomised to | | | | |
| | metoprolol (n=1045) | | diuretic (n=1052) | | Difference | metoprolol (n=535) | | diuretic (n=524) | | Difference |
| | n | rate* | n | rate* | % | n | rate* | n | rate* | % |
| 842 days | 10 | 4.2 | 11 | 4.6 | -8 | 5 | 4.1 | 16 | 13.5 | -70 |
| Median (4.16 years) | 13 | 3.4 | 23 | 6.1 | -44 | 15 | 7.9 | 31 | 16.9 | -53 |
| End of study | 30 | 5.7 | 33 | 6.3 | -10 | 35 | 13.2 | 50 | 19.7 | -33 |

*rate = cases/1000 patient years.

## Claims

1. Use of metoprolol or a salt thereof as an active ingredient in manufacture of a pharmaceutical preparation for improved primary prevention of death in a human patient having mild to moderate hypertension and no known previous hystory of myocardial infarction, brain infarction or cerebral hemorrhage, by long term daily administration of a prophylactically effective dosage of such preparation.

2. Use as claimed in claim 1 wherein said patient is a patient having no known previous history of severe cardiovascular disease.

3. Use as claimed in claim 2 wherein said patient is a patient having uncomplicated mild to moderate hypertension.

4. Use of metoprolol or a salt thereof as an active ingredient in manufacture of a pharmaceutical preparation for reduction of total mortality in a human patient population having mild to moderate hypertension and no known previous hystory of myocardial infarction, brain infarction or cerebral hemorrhage, by long time daily administration of prophylactically effective dosages of metoprolol or a pharmaceutically acceptable salt thereof to said population.

5. Use as claimed in claim 4 wherein said patient population is a population having no known previous history of severe cardiovascular disease.

6. Use as claimed in claim 5 wherein said patient population is a population having uncomplicated mild to moderate hypertension.

7. Use of metoprolol or a salt thereof as an active ingredient in manufacture of a pharmaceutical preparation for primary prevention of death of a human patient for an extended period of time in the order of magnitude of years, such patient having mild to moderate hypertension an no known previous history of myocardial infarction, brain infarction or cerebral hemorrhage, by daily administration of a prophylactically effective dosage of metoprolol or a pharmaceutically acceptable salt thereof to said patient during such extended period.

8. Use of metoprolol or a salt thereof as an active ingredient in manufacture of a pharmaceutical preparation for reducing the total risk of death of a human patient having mild to moderate hypertension and no known previous history of myocardial infarction, brain infaction or cerebral hemorrhage, by long-term daily administration of a prophylactically effective dosage of metoprolol or a pharmaceutically acceptable salt thereof to said patient.

9. Use of metoprolol or a salt thereof as an active ingredient in manufacture of a pharmaceutical preparation for prolonging survival and reducing mortality in a population of adult humans having mild to moderate hypertension and having no known history of myocardial infarction, brain infarction or cerebral hemorrhage by administering at regular intervals of time to said humans a prophylactically effective dose of metoprolol or a pharmaceutically acceptable salt thereof over an extended period of time.

10. Use of metoprolol or a salt thereof as an active ingredient in manufacture of a pharmaceutical preparation for prevention of early death and reduction of total mortality in a population of adult humans having mild to moderate hypertension and having no known history of myocardial infarction, brain infarction or cerebral hemorrhage by administering at regular intervals of time to said humans an effective dose of metoprolol or a pharmaceutically acceptable salt thereof as a prophylactic over an extended period of time.

11. Use of metoprolol or a salt thereof as an active ingredient in manufacture of a pharmaceutical preparation for improving primary prevention of death, over treatment with thiazide diuretics when lowering blood pressure to a predetermined level in a human patient having mild to moderate hypertension and no known previous history of myocardial infarction, brain infarction or cerebral hemorrhage, by long-term daily administration of a prophylactically effective dosage of metoprolol or a pharmaceutically acceptable acid addition salt thereof to said patient.

Additional drugs given to reach treatment goal DBP <95 mm Hg

FIG.1   STUDY DESIGN

## Number of patients and collected patient years in relation to follow-up time

Number of patients (bars)

Patient years cumulative (line)

Median=4.16 years
Mean=5.0 years

Follow-up Years

FIG.2 NUMBER OF PATIENTS AND COLLECTED PATIENT YEARS IN RELATION TO FOLLOW-UP TIME

## Total Mortality

Cumulative numbers

Diuretics (n=1625)

p=0.028

Metoprolol (n=1609)

Follow-up Years

FIG. 3 TOTAL CUMULATIVE MORTALITY IN THE TWO RANDOMISATION GROUPS

Total Mortality
Smoking habits at randomisation

Diuretics
Metoprolol

FIG.4    TOTAL CUMULATIVE MORTALITY IN THE TWO
RANDOMISATION GROUPS ACCORDING TO
SMOKING HABITS AT RANDOMISATION

European Patent
Office

**EUROPEAN SEARCH REPORT**

. Application number

EP  88850353.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | NATIONAL LIBRARY OF MEDICINE (NLM) File Medline, accession no. 84031144, G. N. KARACHALIOS: "A Comparative study of metoprolol and methyldopa in the treatment of hypertension", Int. J. Clin. Pharmcol. Ther. Toxicol. 1983, Sep; 21(9) p. 476-8 * Abstract *  --- | 1-11 | A 61 K 31/135 C 07 C 93/06 |
| X | NATIONAL LIBRARY OF MEDICINE (NLM) File Medline, accession no. 82044518, C. BENGTSSON: "Seven years on a selective beta-blocker-metoprolol. A long-term study of women with arterial hypertension", Ann. Clin. Res. 1981:13, Suppl. 30, p. 7-15 * Abstract *  --- | 1-11 | |
| X | NATIONAL LIBRARY OF MEDICINE (NLM) File Medline, accession no.87123103, J. MILEI, J. LEMUS and E. BERNARDINER: "Ketanserin in the treatment of essential hypertension. A double-blind study against metoprolol and a further long-term open treatment", Acta Cardiol. (Brux) 1986:41(6) p. 427-41 * Abstract *  --- | 1-11 | TECHNICAL FIELDS SEARCHED (Int. Cl.4)  A 61 K 31/135 C 07 C 93/06 |

The present search report has been drawn up for all claims

| Place of search | Date of completion of the search | Examiner |
|---|---|---|
| STOCKHOLM | 17-01-1989 | BERLIN I. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503 03 82

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | NATIONAL LIBRARY OF MEDICINE (NLM) File Medline, accession no. 86000336, J.W. LAMMERS, H.T. FOLGERING and C.L. VAN HERWAARDEN: "Ventilatory effects of long-term treatment with pindolol and metoprolol in hypertensive patients with chronic obstructive lung disease", Br. J. Clin. Pharmacol. 1985, Sep;20(3) p. 205-10 * Abstract * --- | 1-11 | TECHNICAL FIELDS SEARCHED (Int. Cl.4) |